# EUROPEAN PATENT APPLICATION

(11) **EP 1 410 761 A1**
(43) Date of publication of application: **21.04.2004**
(21) Application number: 03256560.8
(22) Date of filing: 17.10.2003
(51) Int. Cl.: A61B 6/00

(54) **Medical image diagnostic system, and information providing server and information providing method**

(30) Priority: 17.10.2002 JP 2002303058; 30.09.2003 JP 2003340753
(71) Applicant: Kabushiki Kaisha Toshiba, Tokyo 105-8001 (JP)
(72) Inventor: Matsumoto, Masanori, Tokyo 105-8001 (JP); Ozawa, Masahiro, Tokyo 105-8001 (JP); Takada, Yoichi, Tokyo 105-8001 (JP); Ikeda, Satoshi, Tokyo 105-8001 (JP)
(74) Representative: Granleese, Rhian Jane

(57) **Abstract**

An X-ray image diagnostic apparatus (101A) in a medical institution (A) is linked to an examination protocol providing server (201) via a wide area network (301). The server (201) has registered examination protocols including information related to X-ray image diagnoses, and searches for and provides an examination protocol in response to a request from each X-ray image diagnostic apparatus (101A). This allows the diagnostic apparatus (101A) to use an examination protocol set in another apparatus in addition to an examination protocol set in itself. In this instance, because the server (201) is furnished with a search function, the diagnostic apparatus (101A) can readily obtain arbitrary information from registered examination protocols.

## Description

The present invention relates to a system providing an examination protocol for a medical image diagnostic apparatus, capable of enhancing the convenience of a medical image diagnostic apparatus being operated by allowing examination protocols used in apparatuses to be shared.

A medical image diagnostic apparatus, such as an X-ray image diagnostic apparatus, has been furnished with a function of presetting an examination protocol. The examination protocol is a data file that determines a series of conditions, including apparatus setting conditions and photographing technique conditions set according to a region to be examined for a diagnosis or the purpose of an examination. With this examination protocol preset function, an examination protocol adequate for an examination can be registered before the examination as a data file in an internal recording device of the apparatus through an input manipulation according to certain setting items. This function can reduce an apparatus setting operation when performing an examination, which in turn makes it possible to achieve an efficient operation by preventing a mistake in the apparatus settings or in the examination manipulations. Besides an examination protocol preset at the shipment from a factory, this function allows the user to add a desired examination protocol at his end.

However, the examination protocol preset function furnished to a conventional medical image diagnostic apparatus merely registers an examination protocol in an internal storage device, and an examination protocol is not shared among medical image diagnostic apparatuses. When a new examination protocol is to be preset, in particular, input manipulations for various items are necessary, and the user is forced to go through a tedious registration work. In addition, there may be a case that the user wishes to use an examination protocol of the same contents as those of the examination protocol used in the past to perform an examination same as the one performed then. Even in such a case, because the examination protocol is not shared among medical image diagnostic apparatuses, when the user uses a different apparatus, he has to preset an examination protocol by referring to various set values from the examination data in the past before the examination.

On the other hand, an attempt has been made in constructing a system in which information obtained when an examination is performed, that is, examination information including medical images, a used quantity of a contrast medium, or a patient identification number or patient-specific information, etc. is saved in a server on a network, thereby allowing a plurality of medical image diagnostic apparatuses to share the information (for example, see JP-A-2001-149354). It should be noted, however, that the system allows the sharing of examination information alone, and has not achieved the sharing of an examination protocol.

As has been described, the conventional medical image diagnostic apparatus is furnished with the examination protocol preset function; however, the function is used merely to register the contents of the conditions, and efficiency remains poor because the user is forced to go through a tedious work when he is going to preset a new examination protocol or an examination protocol of the same contents as those of an examination protocol performed in another medical image diagnostic apparatus.

It is therefore an object of the present invention to provide: a medical image diagnostic system, in which examination protocols are shared among medical image diagnostic apparatuses, so that when various examinations are performed by a medical image diagnostic apparatus, an adequate examination protocol for each examination can be readily obtained and preset in the apparatus; and an information providing server and an information providing method employed in the medical image diagnostic system.

According to first aspect of the present invention, there is provided an examination protocol providing system for providing an examination protocol set in a medical image diagnostic apparatus installed in a medical institution and including a photographing condition corresponding to a content of an examination to be performed on a subject for a diagnosis, comprising: a network to which the medical image diagnostic apparatus is linked; a server linked to the network, and configured to register an examination protocol used in the medical image diagnostic apparatus in connection with examination information indicating a content of an examination, search for an examination protocol that matches with a request condition in response to a request to provide an examination protocol, sent from the medical image diagnostic apparatus, and distribute the searched examination protocol to the medical image diagnostic apparatus that has sent the request; and communication means provided in the medical image diagnostic apparatus, and configured to send the request to provide an examination protocol to the server and receive the examination protocol distributed from the examination protocol providing server via the network.

According to second aspect of the present invention, there is provided an examination protocol providing server for providing an examination protocol set in a medical image diagnostic apparatus installed in a medical institution and including a photographing condition corresponding to a content of an examination to be performed on a subject for a diagnosis, comprising: communication means for communicating with the medical image diagnostic apparatus via a network; registration means for registering an examination protocol used in the medical image diagnostic apparatus in connection with examination information indicating a content of an examination; search means for searching for an examination protocol that matches with a request condition in response to a request to provide an examination protocol, sent from the medical image diagnostic apparatus; and distribution means for distributing the searched examination protocol to the medical image diagnostic apparatus that has sent the request.

According to third aspect of the present invention, there is provided an examination protocol providing method for providing an examination protocol set in a medical image diagnostic apparatus installed in a medical institution and including a photographing condition corresponding to a content of an examination to be performed on a subject for a diagnosis, comprising: registering an examination protocol used in the medical image diagnostic apparatus in connection with examination information indicating a content of an examination; searching for an examination protocol that matches with a request condition in response to a request to provide an examination protocol, sent from a medical image diagnostic apparatus linked via a network; and distributing the searched examination protocol to the medical image diagnostic apparatus that has sent the request.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a block diagram schematically showing a configuration of an X-ray image diagnostic apparatus to which the invention is applied;
FIG. 2 is a block diagram showing a network structure of an X-ray image diagnostic apparatus and a server;
FIG. 3 is a view showing contents of data included in an examination protocol stored in an examination protocol storage portion;
FIG. 4A and FIG. 4B are views showing examples of classifications of examination protocols stored in the examination storage portion;
FIG. 5 is a view showing the examination order of photographing protocols in the examination protocols classified and stored in the examination protocol storage portion;
FIG. 6 is a flowchart showing a switching operation of photographing protocols in an examination protocol;
FIG. 7 is a view showing an operation to make an examination appointment in the X-ray image diagnostic apparatus from an external HIS/RIS;
FIG. 8A and FIG. 8B are views showing examples when options for image comments included in an examination protocol are prepared in the X-ray image diagnostic apparatus;
FIG. 9 is a block diagram showing a configuration of a first embodiment of a medical image diagnostic system according to the invention;
FIG. 10 is a block diagram showing a configuration of a second embodiment of the medical image diagnostic system according to the invention;
FIG. 11 is a block diagram showing a configuration of a third embodiment of the medical image diagnostic system according to the invention; and
FIG. 12 is a flowchart showing a flow of private information deletion processing in a server of a sixth embodiment of the medical image diagnostic system according to the invention.

Embodiments of the invention will now be described in detail with reference to the drawings using an X-ray image diagnostic apparatus for the circulatory system as an example.

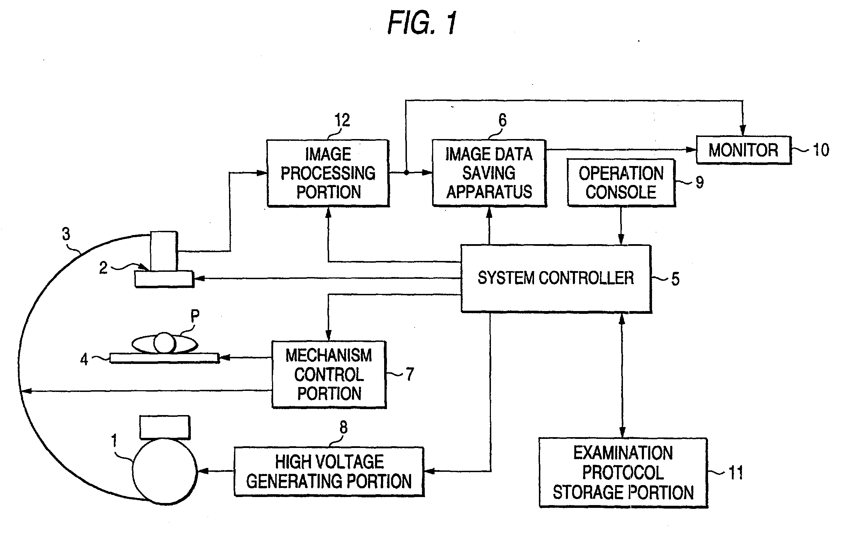

FIG. 1 is a block diagram schematically showing a configuration of an X-ray image diagnostic apparatus to which the invention is applied. Referring to FIG. 1, an X-ray tube 1 irradiates an X-ray upon supply of power from a high voltage generating portion 8, and forms an X-ray photographing portion together with a photographing system 2. The photographing system 2 comprises, for example, a combination of an image intensifier (I.I.) (or a fluorescent screen), an optical system, and a TV camera. Alternatively, the photographing system 2 may comprise an X-ray flat panel detector adopting a direct converting method for converting an X-ray directly into an electrical signal, or an X-ray flat panel detector adopting an indirect converting method for converting an X-ray into light first and then converting the light into an electrical signal, both of which are expected to become the mainstream of X-ray photographing.

The X-ray tube 1 and the photographing system 2 are supported by a C-arm mechanism 3. The C-arm mechanism 3 comprises a rotational mechanism with respect to three or more rotational axes intersecting at right angles with one another, and a sliding mechanism with respect to at least one direction. By driving these mechanisms under control, the C-arm mechanism 3 allows the X-ray tube 1 and the photographing system 2 to take a free photographing posture with respect to a subject P laid on a movable diagnostic table of a diagnostic table mechanism 4. A mechanism control portion 7 controls motions of mechanism portions of the apparatus, including the C-arm mechanism 3 and the diagnostic table mechanism 4.

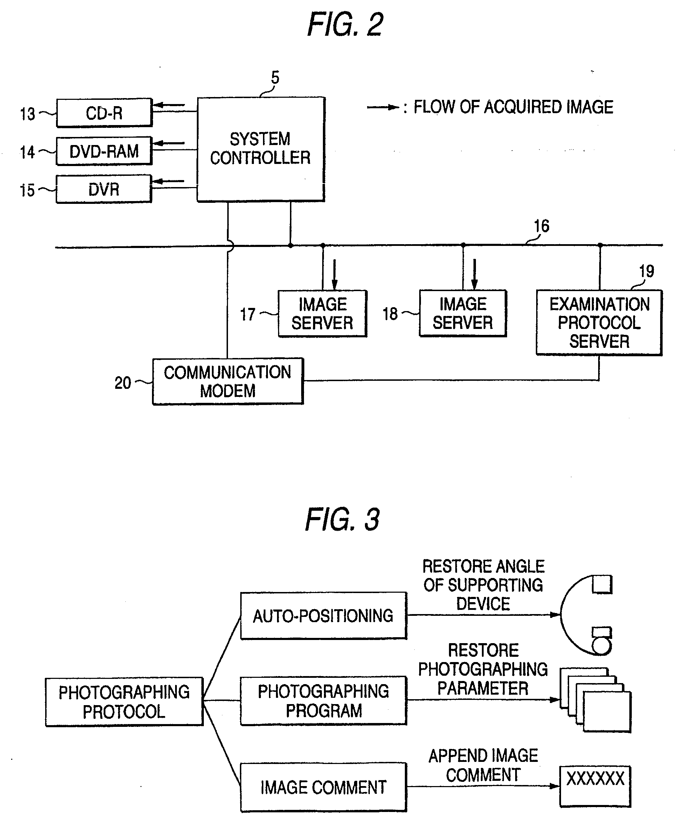

Image data of the subject P obtained by the photographing system 2 through X-ray exposure is sent to an image processing portion 12, and subjected to various kinds of image processing including subtraction processing unique to the circulatory system in addition to analog-to-digital conversion, affine transformation, spatial filtering, temporal filtering, and gamma correction. The image data processed in the image processing portion 12 is displayed on a monitor 10 while being sent to an image data saving apparatus 6 to be saved therein. As shown in FIG. 2, the image data saving apparatus 6 accommodates various kinds of storage medium, such as a CD-R 13, a DVD-RAM 14, and a DVD-R 15, and is also furnished with a function of transferring the image data via a LAN 16 to be stored in image servers 17 and 18 in a medical institution having installed the apparatus.

A system controller 5 controls the photographing system 2, the image processing portion 12, the image data saving apparatus 6, the monitor 10, the mechanism control portion 7, and the high voltage generating portion 8 as a whole. In response to an input manipulation on an operation console 9 for processing to be carried out, the system controller 5 reads out a corresponding examination protocol from an examination protocol storage portion 11, and controls respective components based on the contents of the examination protocol. Also, in response to a registration input manipulation on the operation console 9, the system controller 5 performs registration processing, such as addition and amendment of an examination protocol in the examination protocol storage portion 11. Further, as shown in FIG. 2, the system controller 5 is furnished with a function of uploading/downloading an examination protocol to/from an examination protocol server (local server) 19 in a medical institution having installed the apparatus via a LAN, and a function of uploading/downloading an examination protocol to/from an examination protocol providing server described below through a communication modem 20 via a wide area network outside the medical institution.

An examination protocol may be uploaded or downloaded in a unit of a photographing protocol, which is an examination protocol in a narrow sense. Also, the examination protocol server 19 may be linked to the communication modem 20, so that the examination protocol server 19 per se uploads/downloads an examination protocol or a photographing protocol to/from an examination protocol providing server described below.

The examination protocol storage portion 11 stores a plurality of examination protocols. Each examination protocol is made of one or more photographing protocol, which is an examination protocol in a narrow sense, setting data of a UI (user interface), which is a data group used commonly during a series of examinations, auto-positioning data used to restore an angle of a supporting device, data defining an automatic archive method of an acquired image, photographing instruction information (text information indicating an instruction, such as precautions for the operator), etc.

As shown in FIG. 3, a photographing protocol includes photographing program data for restoring a photographing parameter and an image comment, and in the case of a photographing protocol in which a photographing direction is of great importance, the photographing protocol is correlated with the auto-positioning data used to restore an angle of the supporting device. Although it is not shown in the drawing, the photographing protocol further includes data related to the saving of image data, parameters for image processing, etc.

In other words, an examination protocol is defined as a parameter set that is set in the apparatus when a series of examinations are performed for a patient, and for example, an examination protocol for an examination of head angiography for a patient means the entire group of examination programs, such as a DSA photographing program and a rotation DSA photographing program.

Meanwhile, a photographing protocol is defined as a data set (a data set for photographing techniques) for respective photographing programs in an examination protocol, and for example, a photographing protocol in an examination protocol for an examination of head angiography for a patient means a data set (rotation starting position information, rotation ending position information, an arm rotation rate, a photographing rate, etc.) in a rotation DSA photographing program, which is one of the photographing programs in the photographing protocol.

Also, in the case of different photographing protocols, for example, in the case of a head rotation DSA photographing protocol and an abdomen rotation DSA photographing protocol, each item of data in their respective data sets (rotation starting position information, rotation ending position information, an arm rotation rate, a photographing rate, etc.) has a different recommended value.

It should be noted that one examination protocol is used for one examination, and the photographing protocols defined in the examination protocol are switched as needed.

The auto-positioning data is the data related to a photographing posture of an X-ray photographing system (an angle and a quantity of sliding of a movable portion in the X-ray photographing system) adjusted by the C-arm mechanism 3. The photographing program data for restoring a photographing parameter is the photographing condition data related to photographing conditions (a tube voltage, a tube current, an automatic exposure level, a field for natural light, a quantity of a contrast medium to be injected, an injection rate of a contrast medium, photographing techniques, such as biplane, stereography, and subtraction, a photographing rate at the time of rapid sequence photographing, a photographing time, etc. during a series of operations from fluoroscopy to photographing) when X-ray photographing is performed by the X-ray photographing system 2. Also, the image data may be appended with image comment data, such as the name of a hospital, photographing conditions, and patient attributions.

As has been described, an examination protocol includes set values for all or most of the items the operator has to set in the apparatus when performing a series of operations needed for an examination, such as the setting of a photographing posture, the setting of photographing conditions, the appending of image comments (image accompanying information) to image data, and the saving of image data. Hence, the operator can readily set all or most of a number of setting items that need to be set by the operator when performing a series of examination operations, by a manipulation procedure involving fewer operations, such as a single action of selectively specifying a desired examination protocol item on the operation console 9.

In other words, setting a large number of items in the same manner as in the last time may increase the risk of making a setting mistake, and storing the set values of a large number of items at the operator's end or recording the set values by writing on examination sheets or the like is impractical. On the contrary, by collecting the setting item data needed to perform a series of examination operations into an examination protocol and saving the data as examination protocol items as described above, the setting operation can be simplified markedly. Further, when the operator wishes to perform an examination in the same apparatus state as that in the last examination, he can readily restore the apparatus state.

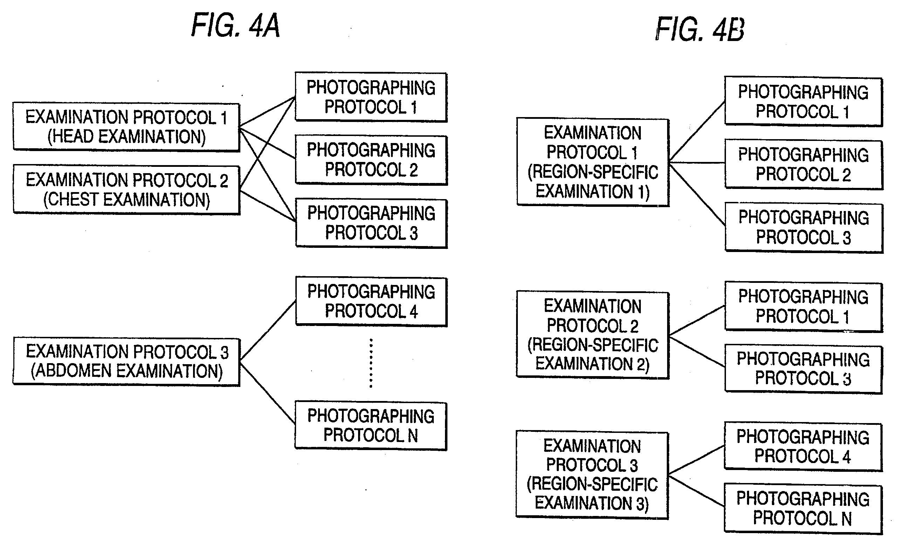

Incidentally, the photographing protocol is correlated with a single examination protocol in the above configuration. However, a method of correlating the photographing protocol to a plurality of examination protocols, such as those for the head, chest, and abdomen, as shown in FIG. 4A and FIG. 4B, is adopted in combination with the configuration described above. According to this method, by merely choosing an examination protocol for a target region to be examined, options are narrowed down to the least necessary photographing protocols correlated with the selected examination protocol. A list of the narrowed down photographing protocols is displayed, so that the operator selectively specifies a desired photographing protocol on the list. This makes it possible to advance an examination by switching to an adequate photographing protocol efficiently.

The correlation between an examination protocol and photographing protocols may adopt a link structure as shown in FIG. 4A or a hierarchical structure as shown in FIG. 4B.

In addition, although it is not shown in the drawings, an examination protocol may be created for each operator (physician). Also, operator (physician) information may be added to data included in an examination protocol. In this case, by choosing an operator when the apparatus is used, options are narrowed down to examination protocols including the operator (physician) information and displayed on a list, so that the operator can selectively specify a desired examination protocol. This allows the operator to preset a desired examination protocol by choosing the examination protocol efficiently from the examination protocols registered in the system.

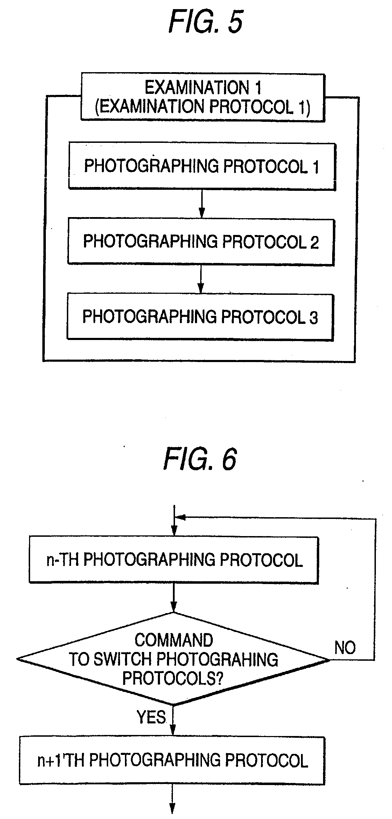

The operator advances an examination by switching a plurality of photographing protocols during a series of examinations. The operator may specify a plurality of photographing protocols collectively at a time to perform specified photographing operations successively. In this case, as shown in FIG. 5, the controller 5 saves the photographing protocol specifying order by the operator, and switches the photographing protocols according to the order thus saved when the examinations are performed. Alternatively, the controller 5 may switch the photographing protocols not by the specified order but according to an overall flow of examinations based on photographing efficiency or the like.

As shown in FIG. 6, this switching is performed in response to a switching command inputted from the operator via the operation console 9. Alternatively, instead of a switching command or using a switching command as an option, a photographing protocol may be switched to the following photographing protocol when the completion of photographing is judged. Further, the switching of photographing protocols may be performed automatically in association with a particular operation, such as a photographing ON/OFF operation. This makes it possible to omit a job of specifying a next photographing protocol from those in an examination protocol each time a photographing operation is completed. Also, this offers an advantage in that no examination is omitted because examinations can be advanced in the same flow as in the last time.

The operator can add, delete, and amend items or the contents of an examination protocol as needed via the operation console 9. Also, the operator can display the contents of the specified examination protocol on the monitor 10 for confirmation. The operator can amend the contents of the examination protocol as needed while he is confirming the contents. It goes without saying that the controller 5 performs an examination according to the amended examination protocol.

Basically, the operator specifies an examination protocol via the operation console 9. At the same time, given that the apparatus is incorporated into a HIS (hospital information system) linking computers inside the hospital to the front desk, a treatment department, an examination department, and further to a PACS (picture archiving and communication system) via a LAN, or into a RIS (radiology information system) inter-linking computers within the department of radiology via a LAN, then the operator is able to specify an examination protocol as an examination appointment from an external terminal within the LAN.

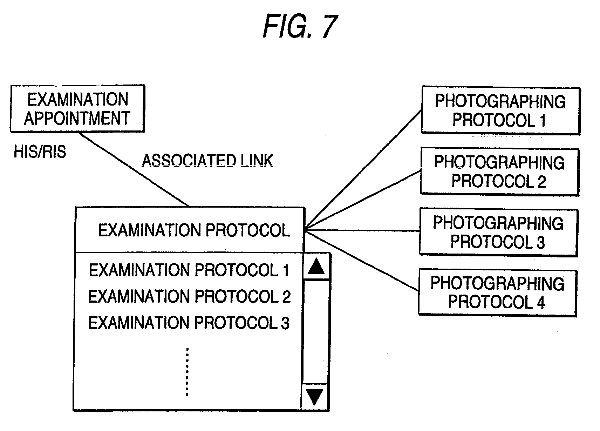

For example, as shown in FIG. 7, when there is a request for an examination appointment from an external terminal, the controller 5 returns a list of examination protocols matching with the request, so that the operator can input an examination appointment to the apparatus (system) from the external terminal with reference to the list. The system can therefore correspond to an on-line examination appointment swiftly.

As has been described, an examination protocol includes image comment data together with the photographing condition data and the posture data. The image comment includes, besides the essential items the examiner or the like has to write in separately, many items related to almost formalized bibliographic matters, such as the name of hospital, a radiographer, an examiner, and a photographing method. Hence, image comments in various forms of these almost formalized items are prepared in advance, so that the examiner or the like can selectively use one of these forms. This enhances the work efficiency, because the examiner or the like only has to input individual items to complete the image comment.

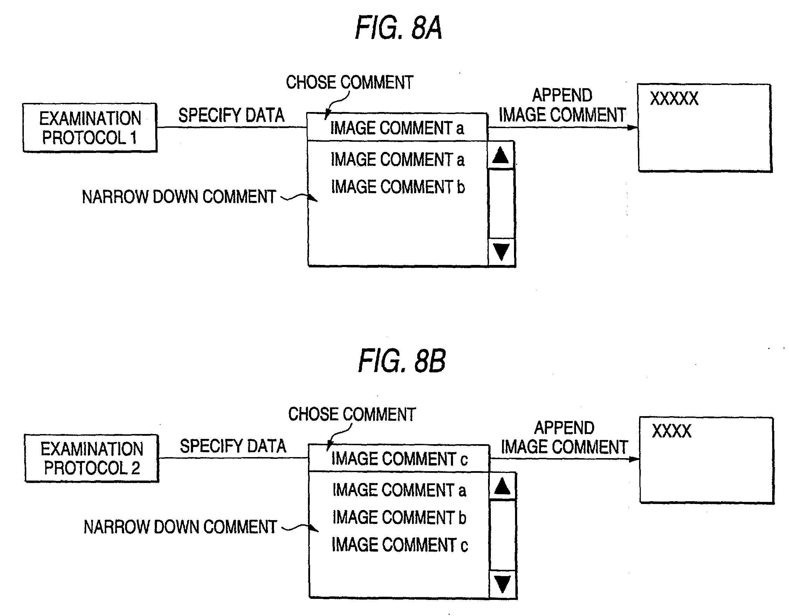

To this end, N kinds of image comments related to various kinds of data in an examination protocol are prepared in advance, and when the photographing condition data or the posture data in the examination protocol is specified, the image comments are narrowed down to M kinds (M<N) as shown in FIG. 8A or FIG. 8B. Further, the image comment data corresponding to the M kinds of image comments is prepared in advance, so that the image comment data is automatically appended to the image data when the operator chooses an arbitrary image comment on a display list showing the image comments narrowed down to M kinds. By incorporating this narrowing down work, efficiency in creating the image comments can be enhanced further.

In connection with enhancement of work efficiency, the choosing operation of an examination protocol is made easier in this embodiment by showing a plurality of examination protocol buttons on a display screen of the operation console 9 in a list of, for example, a GUI (graphic user interface) mode, and thereby allowing the operator to specify an arbitrary examination protocol by an input of a display button choosing operation. It is more effective to reorder the alignment of these buttons according to the purpose of an examination or frequency of use by the controller 5. The items in a photographing protocol can be reordered in the same manner as the examination protocols.

In a case of an examination using an injector system by which a contrast medium is injected automatically, it is configured in such a manner that injector control parameters, such as an injection rate of a contrast medium and a quantity of a contrast medium to be injected, can be written into the photographing condition data of the photographing protocol. In a contrast examination, it is important to make a diagnosis by comparing contrast images each being produced at the same timing, and the configuration described above can enhance the restorability in this regard.

The photographing protocol and the photographing conditions used during the examination are recorded as an examination record in a database for each patient. Hence, on the assumption that the same examination will be performed repetitively for the same patient, information as to a change in the position of the diagnostic table mechanism 4 or the C-arm mechanism 3 made manually during the last examination is written into the examination record, and the controller 5 is furnished with a function of automatically customizing an examination protocol chosen prior to the examination to the setting contents specific to the patient with reference to the examination record. By allowing the operator to choose the examination protocol customized by this function, it is possible to perform an examination under the same apparatus conditions as those in the last photographing operation.

In general, the photographing conditions during the examination are recorded as being automatically appended to the acquired image data according to a particular standard. Hence, the controller 5 is furnished with a function of automatically customizing a photographing protocol with reference to the information appended to the image data. By allowing the operator to choose the photographing protocol customized by this function, it is possible to set the X-ray image diagnostic apparatus in the same conditions as those in the last photographing operation.

There is an occasion that the operator refers to an image taken by another apparatus different from the X-ray diagnostic apparatus to be used in the current examination. Image data is normally in a common data format, such as DICOM. Hence, the controller 5 is furnished with a function of customizing a photographing protocol based on the information appended to the image data. By allowing the operator to choose the photographing protocol customized by this function, the settings of a part of functions of the apparatus, for example, the position of the supporting device or X-ray exposing conditions, can be set correspondingly in another apparatus different from the apparatus that has produced the image being referred to, by a single manipulation.

Embodiments of the medical image diagnostic system according to the invention allowing the X-ray image diagnostic apparatuses to share examination protocols preset in the apparatuses will now be described concretely.

### (First Embodiment)

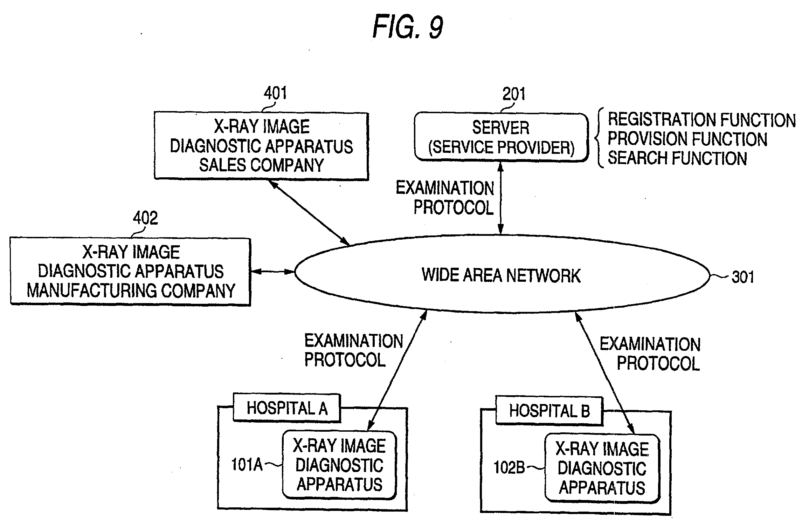

FIG. 9 is a block diagram showing a first embodiment of the medical image diagnostic system according to the invention.

The system of this embodiment is constructed by linking X-ray image diagnostic apparatuses 101A and 101B respectively installed in a plurality of medical institutions (hospitals or the like) A and B to a server 201 used to provide examination protocols, via a wide area network 301. The system is configured to register examination protocols including information related to X-ray image diagnoses in the server 201, so that an examination protocol is provided to each of the X-ray image diagnostic apparatus 101A and 101B from the server 201.

The wide area network 301 comprises the Internet, a telephone line, a network for cellular phones, etc., through which electronic data can be transferred.

As shown in FIG. 2, each of the X-ray image diagnostic apparatuses 101A and 101B is provided with the communication modem 20 to establish a link with the wide area network 301, and is furnished with a function of uploading an examination protocol used when an examination or a treatment was performed to the server 201 as well as a function of downloading a desired examination protocol from the server 201.

The server 201 is provided with a communication modem (not shown) to establish a link with the wide area network 301, and is furnished with a function of registering examination protocols uploaded from the X-ray image diagnostic apparatuses 101A and 101B, a function of providing a corresponding examination protocol to the X-ray image diagnostic apparatus that has sent a download request, and a function of searching for an examination protocol matching with the contents of a download request through registered examination protocols.

By constructing the system as described above, it is possible to provide a service of allowing a plurality of X-ray image diagnostic apparatuses 101A and 101B to share their own examination protocols with the use of the server 201. Each diagnostic apparatus then becomes able to use an examination protocol set in another apparatus in addition to the examination protocol set in itself. In this instance, because the server 201 is furnished with the search function, each diagnostic apparatus can readily obtain an adequate examination protocol among the registered examination protocols in the server 201 by sending a download request to the server 201 together with information indicating the content of an examination, such as a keyword.

### (Second Embodiment)

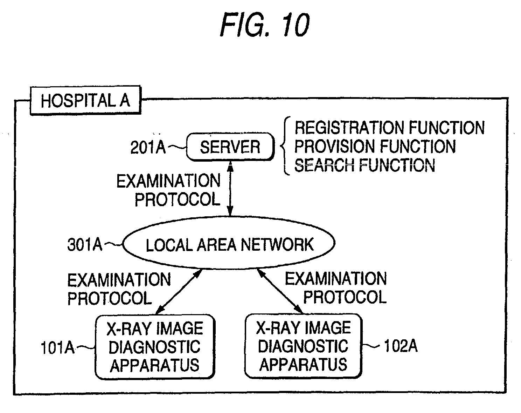

FIG. 10 is a block diagram showing a second embodiment of the medical image diagnostic system according to the invention.

The system of this embodiment is an example when the system of the first embodiment is constructed within a medical institution (hospital or the like). Herein, the system is constructed by installing a server 201A used to save examination protocols including information related to X-ray image diagnostic apparatuses 101A and 102A within a medical institution A, and inter-linking a plurality of X-ray image diagnostic apparatuses 101A and 102A within the medical institution A via a local net work (LAN) 301A.

By constructing the system in this manner, it is possible to provide a service of allowing a plurality of X-ray image diagnostic apparatuses 101A and 102A within the medical institution A to share examination protocols.

### (Third Embodiment)

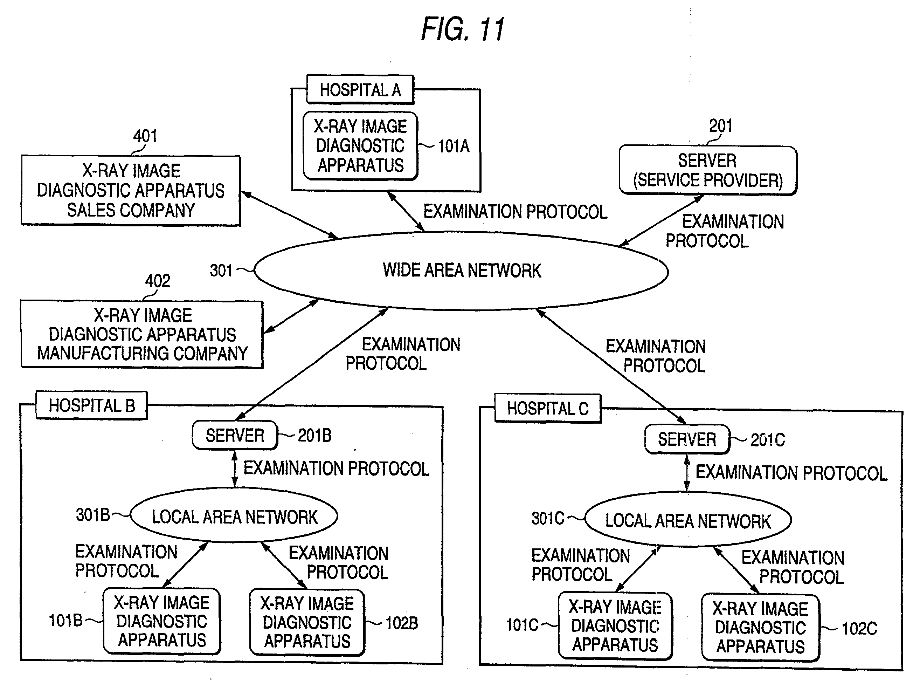

FIG. 11 is a block diagram showing a third embodiment of the medical image diagnostic system according to the invention.

The system of this embodiment is constructed by combining the first embodiment and the second embodiment. Referring to FIG. 11, medical institutions (hospitals or the like) A, B, and C are linked to a wide area network 301, and a server 201 used to save examination protocols including information related to X-ray image diagnostic apparatuses 101A, 101B, 102B, 101C and 102C installed in the medical institutions A, B, and C is also linked to the wide area network 301.

Herein, the medical institution A directly links the X-ray diagnostic apparatus 101A to the wide area network 301 via a communication modem (not shown); on the contrary, the medical institutions B and C link the servers 201B and 201C, which accommodate a plurality of X-ray image diagnostic apparatuses 101B, 102B, 101C, and 102C via local networks 301B and 301C, to the wide area network 301 through their respective communication modems (not shown).

By constructing the system in this manner, for example, the X-ray image diagnostic apparatus 101B becomes able to share examination protocols in all the X-ray image diagnostic apparatuses by allowing the server 201B in the medical institution B to upload/download an examination protocol to/from the server 201 on the wide area network 301, the server 201C in the medical institution C, or the X-ray image diagnostic apparatus 101A in the medical institution A, and by allowing the X-ray image diagnostic apparatus 101B in the medical institution B to upload/download an examination protocol to/from the server 201B in the medical institution B.

Hence, according to this system, it is possible to provide a service of allowing a plurality of X-ray image diagnostic apparatuses installed in respective medical institutions to share examination protocols within each medical institution, while at the same time, it is also possible to provide a service of allowing a plurality of medical institutions to share examination protocols set in the X-ray image diagnostic apparatuses.

### (Fourth Embodiment)

A fourth embodiment of the medical image diagnostic system according to the invention will now be described.

The use of a network is the premise in the first through third embodiments. However, there is an X-ray image diagnostic apparatus that cannot be linked to a network. Also, there may be a case that a network is interrupted.

Hence, in the system of this embodiment, examination protocols are delivered between the server and the X-ray image diagnostic apparatus by means of an electronic saving medium, such as a CD-R/RW, a DVD-RAM/R/RW, and an IC memory, thereby achieving a function equivalent to a downloading/uploading operation through communications.

This service allows an X-ray image diagnostic apparatus to share examination protocols even when it is not linked to a network.

### (Fifth Embodiment)

A fifth embodiment of the medical image diagnostic system according to the invention will now be described.

The system of this embodiment correlates examination information, such as images and comments obtained from the examination actually performed according to a examination protocol, with the examination protocol and appends the former to the latter in the configuration of any of the first through third embodiments, thereby providing a service of allowing the apparatuses to share examination protocols and examination information.

According to this system, because the examination protocol is obtained together with the examination information appended when the examination was performed, whether performing the obtained examination protocol is adequate or not can be readily understood.

### (Sixth Embodiment)

A sixth embodiment of the medical image diagnostic system according to the invention will now be described.

In the fifth embodiment, there may be a case where the examination information includes private information, such as a patient ID and the name of a patient identifying the patient. Disclosing the information to many medical institutions while the private information being appended raises a concern in terms of protecting the privacy of the patient. It should be noted, however, that when the examination information is used to merely check what kinds of images are acquired by performing an examination according to the examination protocol, there will be no problem if the private information of the patient is deleted.

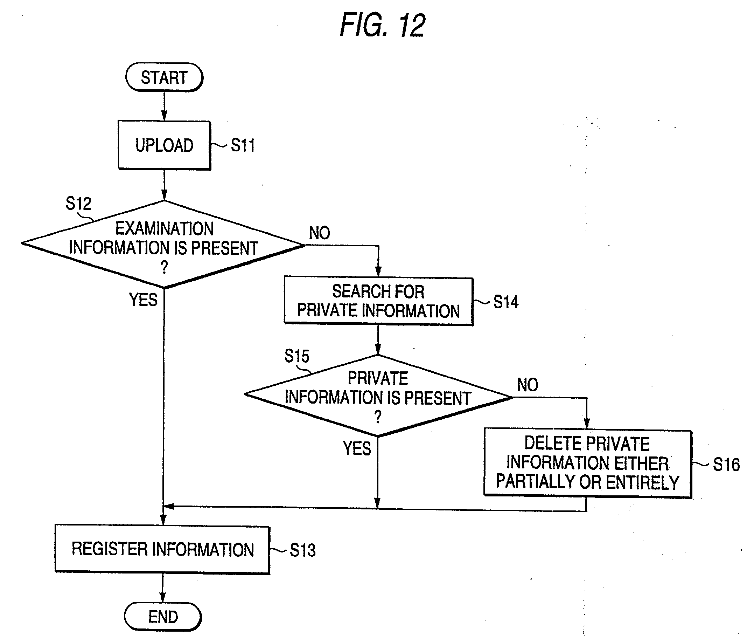

Hence, in the system of this embodiment, the server 201 follows the procedure as shown in FIG. 12. That is, when an examination protocol is uploaded (Step S11), the server 201 judges whether the examination information is appended to the examination protocol (Step S12). Upon judging the absence of the examination information, the server 201 registers the examination protocol intact (Step S13), and upon judging the presence of the examination information, the server 201 searches for the private information (Step S14). The server 201 judges whether the private information is present from the search result (Step S15). Upon judging the absence of the private information, the server 201 registers the examination protocol intact (Step S13), and upon judging the presence of the private information, the server 201 deletes the private information either partially or entirely in terms of protecting the privacy of the patient (Step S16), and then performs the registration processing (Step S13).

It should be appreciated that, in a case where the examination protocol includes the private information, the private information is also searched for and deleted from the examination protocol.

As has been described, according to the system of this embodiment, the private information is searched for at the time of registration and the information is deleted either partially or entirely. This enables the sharing of examination protocols including the examination information while protecting the privacy of the patient. In this instance, even when the uploading apparatus does not delete or fails to delete the private information, the downloading apparatus deletes the private information, which can reduce the work burden on the uploading apparatus.

### (Seventh Embodiment)

A seventh embodiment of the medical image diagnostic system according to the invention will now be described.

In the system of this embodiment, a service provider manages the server 201 on the wide area network 301 in the configuration of the third embodiment. The service provider provides a service of searching through examination protocols registered in the servers 201B and 201C installed in a plurality of medical institutions B and C with the use of the server 201. Also, when examination protocols are downloaded across a plurality of medical institutions, the service provider provides a service of enabling safe download, for example, by authenticating the user identity and ensuring the security.

For example, assume that the medical institution A is registered as a user with the service provider that manages the server 201. Then, when the user wishes to use an examination protocol on the X-ray image diagnostic apparatus 101A in the medical institution A, the user accesses the server 201 and has his user identity authenticated, after which he requests a search by the keyword or the like. Upon receipt of the request, the service provider searches for a corresponding examination protocol through the examination protocols registered in the servers 201B and 201C in the other medical institutions B and C on behalf of the user, and notifies the medical institution A of the search result.

As has been described, according to the system of this embodiment, because the service provider manages the server 201, the user can readily find and obtain the optimal examination protocol without the need to make a search at his end. Also, by authenticating the user identity for each access made to the server 201, unauthorized search or download of the information by any party other than the registered users is inhibited, and the security can be thereby ensured. In this case, an apparatus-specific ID pre-assigned to the X-ray image diagnostic apparatus may be used for authentication of the user identity.

### (Eighth Embodiment)

An eighth embodiment of the medical image diagnostic system according to the invention will now be described.

In the system of this embodiment, a service provider manages the server 201 on the wide area network 301 in the configuration of the first or third embodiment. The service provider receives at the server 201, either with or without charge, the provision of examination protocols and the examination information from the servers or the X-ray image diagnostic apparatuses in the respective medical institutions A, B, C, and so forth. The service provider saves the examination protocols and the examination information in the server 201, and registers keywords indicating the characteristics of examinations in connection with the examination protocols and the examination information.

Upon access to the server 201, the service provider first authenticates the user identity. When the user identity is authenticated, the service provider accepts from the user a search request together with the key words, such as the case, the examination name, the organ examined, the name of the medical institution having performed the examination, the name of the examiner, and the name of the X-ray image diagnostic apparatus used for the examination. The service provider then searches for an examination protocol and examination information with these key words, and notifies the user of a list of the corresponding examination protocol(s) and examination information. When the user requests any or all of the examination protocol(s) and examination information on the list, the service provider allows the user to download the corresponding examination protocol(s) and examination information.

According to the system configuration described above, the apparatus operator is able to download an examination protocol and examination information into an X-ray image diagnostic apparatus in a medical institution (user) having concluded the service contract from the server of the service provider before he starts the examination using the X-ray image diagnostic apparatus. Also, before the apparatus operator starts an examination, he is able to choose the optimal device or the optimal examination method with reference to the examination information of the same case(s) in the past or is further able to simulate an examination while observing the X-ray images. In addition, by performing an examination according to the downloaded examination protocol at the time of examination, there can be expected advantages in reducing labors otherwise needed for the apparatus settings before and during the examination. These advantages are particularly useful in an examination for a rare case.

### (Ninth Embodiment)

A ninth embodiment of the medical image diagnostic system according to the invention will now be described.

In the system of this embodiment, a service provider manages the server 201 on the wide area network 301 in the configuration of the first or third embodiment. The service provider provides, with the use of the server 201, a service of allowing the user to register a recommended examination protocol in connection with the purpose of an examination. In addition, the service provider provides a service of allowing the user to register a recommended examination protocol in connection with a particular medical institution or group. When an examination protocol is registered, the service provider authenticates the identity of the user, so that only the authenticated user is allowed to register an examination protocol.

In this instance, the service provider requests examination information needed to indicate the examination contents from an examination protocol provider. For example, when a user has improved an examination protocol by devising a photographing technique such that can reduce a used quantity of a contrast medium or a quantity of X-ray exposure, or a GUI enhancing the ease of use during an examination, the user is allowed to register the examination protocol thus improved in the server as a recommended examination protocol. The examination protocols thus registered are classified into those disclosed only to particular medical institutions and groups, and to those disclosed to an unspecified number of medical institutions; however, the examination protocols are disclosed after the identity of the user is authenticated.

According to the system configured as described above, the respective medical institutions having concluded the service contract are allowed to perform an examination by downloading a recommended examination protocol at the time of examination, and there can be expected advantages in improving the examination throughput and in enhancing the QOL (quality of life: quality of post-operation life) of the patient.

### (Tenth Embodiment)

A tenth embodiment of the medical image diagnostic system according to the invention will now be described.

In the system of this embodiment, the service provider provides, in the configuration of the ninth embodiment, a service of automatically downloading an examination protocol to a plurality of registered X-ray image diagnostic apparatuses when an examination protocol registered in the server 201 managed by the service provider is updated. Alternatively, the service provider provides a service of automatically downloading an examination protocol only when an examination protocol is updated by a particular physician or medical institution.

By constructing the system in this manner, each X-ray image diagnostic apparatus is always able to obtain the latest examination protocol, which enables the more appropriate settings and diagnosis.

### (Eleventh Embodiment)

An eleventh embodiment of the medical image diagnostic system according to the invention will now be described.

In the system of this embodiment, the service provider provides, in the configuration of the ninth embodiment, a service of automatically maintaining examination protocols in a plurality of registered X-ray image diagnostic apparatus in the same state as that of an examination protocol recoded in a particular X-ray image diagnostic apparatus.

According to this system, in a case where the same patient is treated in a plurality of medical institutions, or by a different diagnostic apparatus installed in the same medical institution, the apparatus to be used can be readily set in an adequate state.

### (Twelfth Embodiment)

A twelfth embodiment of the medical image diagnostic system according to the invention will now be described.

In the system of this embodiment, a service provider manages the server 201 on the wide area network 301 in the configuration of the first or third embodiment. As shown FIG. 1 or FIG. 3, the service provider provides, with the use of the server 201, a service of allowing a particular X-ray image diagnostic apparatus sales company 401 or X-ray image diagnostic apparatus manufacturing company 402 on the wide area network 301 to share an examination protocol involving manipulation means in a particular X-ray image diagnostic apparatus and examination information including images acquired through this manipulation, among examination protocols in a plurality of registered X-ray image diagnostic apparatuses.

According to the system configuration described above, for example, on an occasion to demonstrate the use of the apparatus, the X-ray image diagnostic apparatus sales company or the X-ray image diagnostic apparatus manufacturing company having concluded the service contract is able to explain the operation while allowing the user to observe X-ray images acquired when an examination is performed according to the examination protocol, by downloading an examination protocol and the examination information in the apparatus without actually performing the examination. Also, by requesting the X-ray image diagnostic apparatus sales company or the X-ray image diagnostic apparatus manufacturing company to verify an examination protocol and the examination information registered in the server, the quality of the registered information can be upgraded.

### (Thirteenth Embodiment)

A thirteenth embodiment of the medical image diagnostic system according to the invention will now be described.

In the system of this embodiment, the X-ray image diagnostic apparatus sales company 401 or the X-ray image diagnostic apparatus manufacturing company 402 saves examination protocols and the examination information in the server 201 in the configuration of the twelfth embodiment. According to this system, for example, even when an X-ray image diagnostic apparatus becomes unable to return the examination protocol and the examination information to the initial setting, the X-ray image diagnostic apparatus can download the examination protocol and the examination information of the initial setting from the server.

### (Fourteenth Embodiment)

A fourteenth embodiment of the medical image diagnostic system according to the invention will now be described.

In the system of this embodiment, vender terminals 401 and 402 respectively installed in the X-ray image diagnostic apparatus sales company and the X-ray image diagnostic apparatus manufacturing company are linked to the server 201 via the network 301 in the twelfth or thirteenth embodiment, and the service provider provides a service of allowing the X-ray image diagnostic apparatus sales company or the X-ray image diagnostic apparatus manufacturing company to download examination protocols to the X-ray image diagnostic apparatuses 101A and 101B from the server 201 when delivering the apparatuses 101A and 101B to the medical institutions. This provides advantages in that the medical institutions are able to use an X-ray image diagnostic apparatus with the latest examination protocol as soon as the apparatus is installed.

### (Fifteenth Embodiment)

A fifteenth embodiment of the medical image diagnostic system according to the invention will now be described.

In the system of this embodiment, a service provider manages the server 201 on the wide area network 301 in the configuration of the first or third embodiment. The service provider provides, with the use of the server 201, a service of allowing the apparatus operator (physician) having concluded the service contract to register an examination protocol of his own.

According to the system, when the apparatus operator performs examinations in a plurality of medical institutions, the apparatus operator is able to download the examination protocol in the X-ray image diagnostic apparatuses installed in the respective medical institutions from the server, and perform an examination according to the downloaded examination protocol. The apparatus operator is thus able to use any X-ray image diagnostic apparatus according to the same customized manipulation procedure without having to go through a tedious setting operation that is otherwise needed for each apparatus. Hence, there can be expected advantages in preventing an erroneous manipulation or reducing stresses caused by difference in operability, and in allowing the apparatus operator to concentrate on the examination operation.

### (Sixteenth Embodiment)

A sixteenth embodiment of the medical image diagnostic system according to the invention will now be described.

In the system of this embodiment, a service provider manages the server 201 on the wide area network 301 in the configuration of the first or third embodiment. The service provider provides, with the use of the server 201, a service of allowing a plurality of medical institutions to save the examination protocol and examination information at the time of examination in connection with examination history for each patient to be shared.

For example, when a patient has medical checks in different medical institutions in an acute treatment and in a chronic treatment, and a variance with time is to be observed by comparing X-ray images, it is preferable to obtain X-ray images by performing examinations in each medical institution under the same conditions. By performing an examination according to the examination protocol downloaded from the server, each medical institution becomes able to perform an examination under the same conditions as those in the examination in the past; moreover, the labors needed for the setting of the examination conditions can be omitted. Hence, there can be expected advantages in improving the examination throughput.

### (Seventeenth Embodiment)

A seventeenth embodiment of the medical image diagnostic system according to the invention will now be described.

In the system configuration of the first or third embodiment, the service provider manages the server 201 on the wide area network 301. Herein, the service provider concludes a contract related to the provision of a service with each apparatus operator (physician) or each apparatus, and provides the service only to those having concluded the contract.

A medical institution inputs an examination code, which is determined by the clinical path defining a treatment, the contents of an examination, the contents of diet, etc. on the time line for each illness, from the X-ray image diagnostic apparatus, or by inserting a card having the examination code as input data into a card reader in the X-ray image diagnostic apparatus.

Examination code information is then registered on the wide area network 301, and a request to provide an examination protocol is sent from the medical institution (apparatus) to the server.

The server 201 then searches for an examination protocol adequate to the contents of an examination based on the examination code information, and registers the searched examination protocol on the wide area network 301. The request-sender medical institution (apparatus) is thus able to download the examination protocol adequate to the contents of the examination code information determined by the clinical path via the wide area network 301.

By giving a code common to the service providers as the examination code information, and by registering the examination code information on the wide area network together with the examination protocol, the examination protocol can be provided swiftly in a case where an examination protocol having the same examination code information has been registered in another hospital. The user only has to pass an adequate check by the provider and receive an authorization to receive the provision of the examination protocol.

In the case of the local server 201 in the system configuration of the second embodiment, correspondence data between the examination codes and the examination protocols adequate to the contents thereof is set in advance in the local server.

When the medical institution inputs the examination code determined by the clinical path defining the treatment, the contents of an examination, the contents of diet, etc. on the time line for each illness from the X-ray image diagnostic apparatus, or inserts the card having the examination code as input data into the card reader in the X-ray image diagnostic apparatus, the server 201 starts to search for an examination protocol adequate to the examination code information based on the pre-registered information. The X-ray image diagnostic apparatus then downloads the examination protocol extracted as a result of the search.

When the apparatus (hospital) judges that the contents of the examination protocol provided from the server 201 needs amendments, information related to the contents of amendments is fed back to the server 201 via the wide area network 301. The server 201 then updates the contents of the examination protocol to the more accurate contents based on the feedback information. Further, it is arranged in such a manner that the feedback information is received from another hospital via the wide area network, and the updated information can be therefore shared via the network.

As has been described, by inputting the examination code information determined by the clinical path, only the predetermined examination protocol becomes usable.

This forestalls a mistake in parameter settings due to an erroneous manipulation, the lack of knowledge, and misunderstanding, and eliminates a difference in examination levels among the operators, and the medical quality can be thereby maintained at the constant level.

Further, by including an amendment tolerance of each parameter value in the definition of the examination protocol, the medical quality can be maintained within a certain range while the convenience is improved at the same time.

### (Eighteenth Embodiment)

An eighteenth embodiment of the medical image diagnostic system according to the invention will now be described.

In the system configuration of the first or third embodiment, a service provider manages the server 201 on the wide area network 301. Herein, the service provider concludes a contract related to the provision of a service with each apparatus operator (physician) or each apparatus, and provides a service only to those having concluded the contract.

In an X-ray image diagnostic apparatus in a medical institution, a time needed for one patient to perform an examination according to each examination protocol is measured, which is sent to the local server 201 via the wide area network 301 as throughput information, and the data is accumulated in the server 201.

The throughput information is collected from each medical institution having concluded the contract with the service provider, and accumulated as the throughput information of the examination protocol.

Then, for example, upon acceptance of a request to provide the throughput information (time needed for one patient to perform an examination) of a particular examination protocol from a medical institution, the server 201 searches through the accumulated information, and provides the corresponding throughput information to the request-sender medical institution.

In the case of the local server 201 in the system configuration of the second embodiment, a time needed for one patient to perform an examination according to each examination protocol is measured in each X-ray image diagnostic apparatus within the hospital, and the measured times are accumulated in the local server 201 as throughput information.

When an examination is to be performed according to a given examination protocol in the hospital, the server 201 searches for the throughput information of this examination protocol, which allows the operator to know the time needed to perform an examination according to the examination protocol.

It goes without saying that the server may provide the throughput information together with the examination protocol when the examination protocol is provided to a medical institution having concluded the contract.

The server is able to provide at least any one of the average value, the maximum value, the minimum value of the times needed for an examination according to the same examination protocol accumulated as the throughput information.

Moreover, for example, when a hospital having a plurality of X-ray image diagnostic apparatuses sends a request for the throughput information related to a plurality of examination protocols to the server 201, because the server 201 is able to know the relation between the respective X-ray image diagnostic apparatuses and the time needed to perform an examination according to each examination protocol, the server 201 can provide information related to a combination of an examination protocol and an X-ray image diagnostic apparatus to achieve the highest examination efficiency, for example, by figuring out which examination protocol should be performed by which X-ray image diagnostic apparatus to shorten an examination time.

Because the throughput of the overall examination work is now concerned, work efficiency of the overall examination work can be improved.

According to each of the embodiments above, by providing a service of allowing a plurality of X-ray image diagnostic apparatuses to share the examination protocols, it is possible to perform an examination smoothly by reducing the labor of setting an examination protocol before the examination, and by performing the examination according to the optimal examination protocol. Hence, there can be expected advantages in improving the examination throughput.

Also, by providing a service of allowing the apparatuses to share the optimal examination protocol, the patient can receive the examination that best suits to the patient. Hence, there can be expected advantages in improving the QOL of the patient.

In constructing the system as in each of the embodiments above, when the service provider charges the medical institution having concluded the service contract the fixed fees in each month or fees for a quantity of downloaded data, providing the download service of the examination protocols and the examination information can be established as a business.

In each of the embodiments above, an explanation was given to the X-ray image diagnostic apparatus. It should be appreciated, however, that the invention is not limited to the X-ray image diagnostic apparatus, and is applicable to an examination protocol for other types of medical image diagnostic apparatus as well.

The examination protocol providing server is linked to the vender terminal of the medical image diagnostic apparatus via the network, so that the examination protocol providing server allows also the vender of the medical image diagnostic apparatus to share the registered examination protocols, and thereby registers an examination protocol provided from the vender terminal, and sends an examination protocol uploaded from the medical image diagnostic apparatus to the vender terminal. This allows the vender to upload an examination protocol for a medical image diagnostic apparatus newly developed on the vender side, and allows the vender to verify an examination protocol uploaded from the medical image diagnostic apparatus.

The medical institution may inter-link a plurality of hospitals via the local network, so that a plurality of hospitals are allowed to share the downloaded examination protocols.

The examination protocol providing server prepares a group of examination protocols including recommended parameters and photographing directions for each region to be examined, and provides the group of examination protocols when a region to be examined is specified as a request condition. The medical image diagnostic apparatus is thus able to choose an adequate examination protocol as needed, and combines examination protocols when necessary.

The examination protocol providing server authenticates the identity of the user based on identification information pre-assigned to the medical image diagnostic apparatus, and denies the provision of an examination protocol to an apparatus having no access right. This makes it possible to avoid unauthorized use.

Incidentally, an examination protocol to be provided does not necessarily match with the purpose of use or the contents of an examination, and consideration must be given to a case where the provided examination protocol is unusable. However, making the examination protocol unusable unlimitedly makes the service business unachievable. The examination protocol providing server therefore sets a trial period or the number of trials for an examination protocol to be provided, and starts to charge the user when the trail period expires or the number of use exceeds the number of trails. The charges only for an adequate examination protocol can be thus collected from the user, which makes it possible to enhance the service quality.

Upon receipt of a request to provide an examination protocol, the examination protocol providing server judges the type of the request-sender medical image diagnostic apparatus, and presents examination protocols usable in the apparatus as well as examination protocols unusable in the apparatus to the request-sender apparatus. This can avoid an unusable examination protocol from being downloaded, thereby reducing a useless work.

The examination information includes the name of the setter, that is, a person who has set the examination protocol, and when the name of the setter is specified as a request condition, the examination protocol providing server searches for a corresponding examination protocol based on the name of the setter, and provides the searched examination protocol. The user is thus able to download the examination protocol he has uploaded in the past or an examination protocol a famous physician or the like has uploaded.

When a new examination protocol is registered, such as the one in the upgraded version, the examination protocol providing server judges whether this examination protocol is recommendable from the state of use of each user, and upon judging as being recommendable, the examination protocol providing server introduces the new examination protocol to the user. This encourages the user to optimize the apparatus he is using. As a method of charging, the service provider managing the examination protocol providing server may collect fixed fees for a period unit or fees for a quantity of downloaded data from the medical institutions having concluded the service contract. In this instance, when an examination protocol to be provided is set with a trial period or the number of trials, the service provider does not charge for this period nor up to the number of trials. This makes it possible to upgrade the quality of examination protocol providing service.
Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A medical image diagnostic system for providing an examination protocol including a photographing condition corresponding to a content of an examination for a subject, said system **characterized by** comprising:
a network (301) to which a medical image diagnostic apparatus (101A, 102B) is linked; and
a server (201) linked to said network (301), and configured to register an examination protocol for an examination to be performed by said medical image diagnostic apparatus (101A, 102B) in connection with examination information indicating a content of the examination, search for an examination protocol that matches with a request condition in response to a request to provide an examination protocol, sent from said medical image diagnostic apparatus (101A, 102B), and distribute the searched examination protocol to said medical image diagnostic apparatus (101A, 102B) that has sent the request,
wherein said medical image diagnostic apparatus (101A, 102B) sends the request to provide an examination protocol to said server (201) and receives the examination protocol distributed from said server (201) via said network (301).

2. The medical image diagnostic system according to claim 1, **characterized in that**:
said network includes an external network and an internal network of a medical institution;
said medical image diagnostic apparatus is linked to said internal network; and
said server is linked to said external network.

3. The medical image diagnostic system according to claim 2, **characterized in that**:
said medical institution includes a plurality of hospitals;
said internal network inter-links said plurality of hospitals; and
said plurality of hospitals share the examination protocol.

4. The medical image diagnostic system according to claim 2, **characterized by** further comprising:
a local server linked to said internal network and said external network, and configured to send, from said medical image diagnostic apparatus to said server, the request to provide an examination protocol, and send the examination protocol distributed from said examination protocol providing server to said medical image diagnostic apparatus that has sent the request.

5. The medical image diagnostic system according to claim 4, **characterized in that**:
said local server registers examination protocols used in a plurality of medical image diagnostic apparatuses within said medical institution, distributes a corresponding examination protocol in response to a request to a medical image diagnostic apparatus that has sent the request, and, on behalf of said plurality of medical image diagnostic apparatuses, sends a request to provide an examination protocol to said examination protocol providing server and receives the examination protocol.

6. The medical image diagnostic system according to claim 1, **characterized in that**:
said server registers an examination protocol uploaded from said medical image diagnostic apparatus in response to a request to register an examination protocol, sent from said medical image diagnostic apparatus; and
said medical image diagnostic apparatus, via said network, sends the request to register an examination protocol to said server, and upon receipt of a response from said server, uploads an examination protocol set when an examination was performed on the subject to said server, together with examination information indicating a content of the examination.

7. The medical image diagnostic system according to claim 6, **characterized in that**:
said server requests examination information related to an examination protocol to be uploaded, from said medical image diagnostic apparatus that has sent the request to register an examination protocol.

8. The medical image diagnostic system according to claim 6, **characterized in that**:
said server judges whether the examination information includes private information at a time of registration, and upon judging presence of the private information, registers the examination information by one of the following:
by deleting the private information partially;
by deleting the private information entirely; and
by replacing the private information with another information.

9. The medical image diagnostic system according to claim 1, **characterized in that**:
said server registers a keyword indicating a characteristic of the examination protocol from the examination information, and searches for a corresponding examination protocol based on the key word when the keyword is specified as the request condition.

10. The medical image diagnostic system according to claim 1, **characterized in that**:
said server registers an examination purpose for which the examination protocol is recommended as being optimal in connection with the examination protocol, and searches for an examination protocol recommended by the examination purpose when the examination purpose is specified as the request condition.

11. The medical image diagnostic system according to claim 1, **characterized in that**:
said server authenticates an access right when an access is made to said server.

12. The medical image diagnostic system according to claim 11, **characterized in that**:
said server makes authentication based on identification information pre-assigned to said medical image diagnostic apparatus.

13. The medical image diagnostic system according to claim 1, **characterized in that**:
said server automatically distributes an updated examination protocol to a particular medical image diagnostic apparatus when a registered examination protocol is updated.

14. The medical image diagnostic system according to claim 1, **characterized in that**:
said server automatically maintains an examination protocol in a registered medical image diagnostic apparatus in the same state as a state of an examination protocol recorded in a particular medical image diagnostic apparatus.

15. The medical image diagnostic system according to claim 1, **characterized in that**:
the examination information includes a medical image acquired when an examination was performed according to the examination protocol; and
said server distributes the medical image included in the examination information when providing the examination protocol.

16. The medical image diagnostic system according to claim 1, **characterized in that**:
said server registers a customized result of the examination protocol.

17. The medical image diagnostic system according to claim 1, **characterized in that**:
the examination information includes an examination history of the subject; and
said server searches for a corresponding examination protocol based on the examination history when the examination history is specified as the request condition.

18. The medical image diagnostic system according to claim 1, **characterized in that**:
said server is linked to a vender terminal of said medical image diagnostic apparatus via said network, and thereby allows a vender of said medical image diagnostic apparatus also to share a registered examination protocol.

19. The medical image diagnostic system according to claim 18, **characterized in that**:
said server registers an examination protocol provided from said vender terminal, and sends an examination protocol uploaded from said medical image diagnostic apparatus to said vender terminal.

20. The medical image diagnostic system according to claim 1, **characterized in that**:
said medical image diagnostic apparatus is an X-ray photographing apparatus.

21. The medical image diagnostic system according to claim 1, **characterized in that**:
said server prepares a group of examination protocols including recommended parameter and photographing direction for each region to be examined, and distributes the group of examination protocols when a region to be diagnosed is specified as the request condition.

22. The medical image diagnostic system according to claim 1, **characterized in that**:
said server sets one of a trial period and the number of trials in an examination protocol to be distributed.

23. The medical image diagnostic system according to claim 1, **characterized in that**:
upon receipt of the request to provide an examination protocol, said server judges a type of said medical image diagnostic apparatus that has sent the request, and presents examination protocols usable and examination protocols unusable in said medical image diagnostic apparatus.

24. The medical image diagnostic system according to claim 1, **characterized in that**:
the examination information includes a name of a setter of the examination protocol; and
said server searches for a corresponding examination protocol based on the name of the setter when the name of the setter is specified as the request condition.

25. The medical image diagnostic system according to claim 1, **characterized in that**:
said server introduces a recommended examination protocol from a state of use of a user.

26. The medical image diagnostic system according to claim 1, **characterized in that**:
a service provider managing said server charges a medical institution having concluded a service contract one of fixed fees in a period unit and fees for a quantity of downloaded data.

27. The medical image diagnostic system according to claim 26, **characterized in that**:
when an examination protocol to be provided is set with one of a trial period and the number of trials, said service provider does not charge for the period nor up to the number of trails.

28. An information providing server for providing an examination protocol including a photographing condition corresponding to a content of an examination for a subject, said server **characterized by** comprising:
a communication portion configured to communicate with a medical image diagnostic apparatus via a network;
a registration portion configured to register an examination protocol used in said medical image diagnostic apparatus in connection with examination information indicating a content of an examination;
a search portion configured to search for an examination protocol that matches with a request condition in response to a request to provide an examination protocol, sent from said medical image diagnostic apparatus; and
a distribution portion configured to distribute the searched examination protocol to said medical image diagnostic apparatus that has sent the request.

29. An information providing method for providing an examination protocol including a photographing condition corresponding to a content of an examination for a subject, said method **characterized by** comprising:
registering an examination protocol used in a medical image diagnostic apparatus in connection with examination information indicating a content of an examination;
searching for an examination protocol that matches with a request condition in response to a request to provide an examination protocol, sent from a medical image diagnostic apparatus linked via a network; and
distributing the searched examination protocol to said medical image diagnostic apparatus that has sent the request.

30. A medical image diagnostic system for providing an examination protocol including a photographing condition corresponding to a content of an examination for a subject, said system **characterized by** comprising:
a network to which a medical image diagnostic apparatus is linked; and
a server linked to said network, and configured to register a photographing protocol, which is a data set of photographing program in an examination protocol for an examination to be performed by said medical image diagnostic apparatus, search for a photographing protocol that matches with a request condition in response to a request sent from said medical image diagnostic apparatus, and distribute a searched photographing protocol to said medical image diagnostic apparatus that has sent the request,
wherein said medical image diagnostic apparatus sends the request to said server and receives a photographing protocol distributed from said server via said network.

31. The medical image diagnostic system according to claim 30, **characterized in that**:
said server registers the examination protocol in connection with a target region examined; and
when an examination protocol for the target region examined is chosen in said medical image diagnostic apparatus, a photographing protocol correlated with the examination protocol is displayed on a monitor based on the registered information.

32. The medical image diagnostic system according to claim 30, **characterized in that**:
said server registers information related to an operator in connection with the examination protocol; and
when the operator of said medical image diagnostic apparatus is chosen, an examination protocol correlated with the operator is displayed on a monitor based on the registered information.

33. The medical image diagnostic system according to claim 30, **characterized in that**:
said server registers information related to the subject in connection with the examination protocol; and
when the subject who will take an examination by said medical image diagnostic apparatus is chosen, an examination protocol correlated with the subject is displayed on a monitor based on the registered information.

34. The medical image diagnostic system according to claim 30, **characterized by** further comprising:
a controller configured to customize a photographing protocol based on information appended to image data acquired during an examination performed by said medical image diagnostic apparatus.

35. The medical image diagnostic system according to claim 30, **characterized in that**:
said server distributes an examination protocol based on information related to a clinical path inputted from said medical image diagnostic apparatus.

36. The medical image diagnostic system according to claim 30, **characterized in that**:
said server registers information from a medical institution, related to a time needed for one patient to perform an examination according to an examination protocol, and, when distributing the examination protocol, appends thereto the information related to the time needed to perform an examination according to the examination protocol.

37. The medical image diagnostic system according to claim 36, **characterized in that**:
the information related to the time needed for an examination distributed from said server is one of a maximum value, an average value, and a minimum value of times needed for an examination registered in said server.
